# EUROPEAN PATENT APPLICATION

(11) **EP 4 458 249 A1**
(43) Date of publication of application: **06.11.2024**
(21) Application number: 21969883.4
(22) Date of filing: 27.12.2021
(51) Int. Cl.: A61B 1/045

(54) **IMAGE PROCESSING DEVICE, IMAGE PROCESSING METHOD, AND STORAGE MEDIUM**

(71) Applicant: NEC Corporation, Tokyo 108-8001 (JP)
(72) Inventor: SAIGA, Hiroyasu, Tokyo 108-8001 (JP); MAEDA, Naoto, Tokyo 108-8001 (JP); KIMURA, Tatsu, Tokyo 108-8001 (JP); OKUNO, Takayuki, Tokyo 108-8001 (JP)
(74) Representative: Betten & Resch
(86) International application number: PCT/JP2021/048499
(87) International publication number: WO 2023/126999

(57) **Abstract**

The endoscopic image acquisition means acquires an endoscopic image. The polyp detection means detects a polyp area from the endoscopic image. The first estimation means estimates a size of the polyp based on an image of the detected polyp area. The output means outputs an estimation result of the size of the polyp.

## Description

### TECHNICAL FIELD

The present disclosure relates to processing of images relating to an endoscopic examination.

### BACKGROUND ART

When performing treatment of polyp in an endoscopic examination, a doctor selects a treatment instrument according to the polyp size. At this time, the doctor should select the treatment instrument after discerning the polyp in millimeters (mm). However, since the doctor measures the polyp size visually, the measurement in mm units was not enough and it was difficult to judge the treatment method. Patent Document 1 proposes a method of recognizing a target area with high accuracy from an image captured by an endoscopic imaging device.

### PRECEDING TECHNICAL REFERENCES

### PATENT DOCUMENT

Patent Document 1: International Publication WO2021/140602

### SUMMARY

### PROBLEM TO BE SOLVED

However, according to Patent Document 1, it is not always possible to determine the polyp size with high accuracy.

It is an object of the present disclosure to provide an image processing device capable of estimating the size of polyp found in the endoscopic examination with high accuracy.

### MEANS FOR SOLVING THE PROBLEM

According to an example aspect of the present disclosure, there is provided an image processing device comprising:
an endoscopic image acquisition means configured to acquire an endoscopic image;
a polyp detection means configured to detect a polyp area from the endoscopic image;
a first estimation means configured to estimate a size of the polyp based on an image of the detected polyp area; and
an output means configured to output an estimation result of the size of the polyp.

According to another example aspect of the present disclosure, there is provided an image processing method comprising:
acquiring an endoscopic image;
detecting a polyp area from the endoscopic image;
estimating a size of the polyp based on an image of the detected polyp area; and
outputting an estimation result of the size of the polyp.

According to still another example aspect of the present disclosure, there is provided a recording medium storing a program, the program causing a computer to execute processing of:
acquiring an endoscopic image;
detecting a polyp area from the endoscopic image;
estimating a size of the polyp based on an image of the detected polyp area; and
outputting an estimation result of the size of the polyp.

### EFFECT

According to the present disclosure, it is possible to estimate the size of polyp found in the endoscopic examination with high accuracy.

### BRIEF DESCRIPTION OF THE DRAWINGS

[FIG. 1] FIG. 1 is a block diagram showing a schematic configuration of an endoscopic examination system according to a first example embodiment.
[FIG. 2] FIG. 2 is a block diagram showing a hardware configuration of an image processing device according to the first example embodiment.
[FIG. 3] FIG. 3 is a block diagram showing a functional configuration of the image processing device according to the first example embodiment.
[FIG. 4] FIG. 4 is a flowchart of image display processing by the image processing device according to the first example embodiment.
[FIG. 5] FIG. 5 shows a display example of an estimation result of a polyp size.
[FIG. 6] FIG. 6 is a block diagram showing a functional configuration of a modification 1 according to the first example embodiment.
[FIG. 7] FIG. 7 is a block diagram showing a functional configuration of a modification 2 according to the first example embodiment.
[FIG. 8] FIG. 8 is a block diagram showing a functional configuration of an image processing device according to a second example embodiment.
[FIG. 9] FIG. 9 is a flowchart of image display processing by the image processing device according to the second example embodiment.
[FIG. 10] FIG. 10 is a block diagram showing a functional configuration of an image processing device according to a third example embodiment.
[FIG. 11] FIG. 11 is a flowchart of processing by the image processing device according to the third example embodiment.

### EXAMPLE EMBODIMENTS

Preferred example embodiments of the present disclosure will be described with reference to the accompanying drawings.

### <First example embodiment>

### [System configuration]

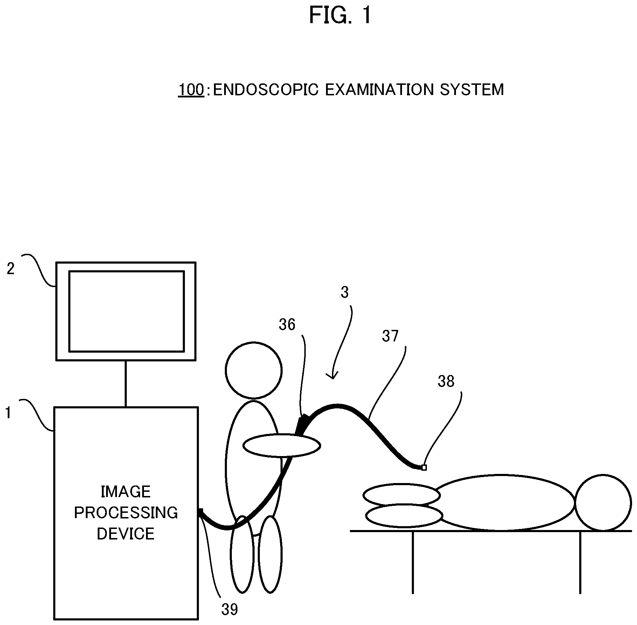

FIG. 1 shows a schematic configuration of an endoscopic examination system 100. At the time of examination (including treatment) using the endoscope, when the endoscopic examination system 100 detects a polyp, the endoscopic examination system 100 estimates whether or not the size of detected polyp is a predetermined size or more and displays the result. Thus, the doctor can judge the treatment method according to the polyp size.

As shown in FIG. 1, the endoscopic examination system 100 mainly includes an image processing device 1, a display device 2, and an endoscope 3 connected to the image processing device 1.

The image processing device 1 acquires an image (i.e., a movie, hereinafter also referred to as an "endoscopic video Ic") captured by the endoscope 3 during the endoscopic examination from the endoscope 3 and displays display data for the check by the examiner of the endoscopic examination on the display device 2. Specifically, the image processing device 1 acquires a moving image of organs captured by the endoscope 3 as an endoscopic video Ic during the endoscopic examination. The image processing device 1 extracts a still image (frame image) from the endoscopic video Ic, and detects the polyp and estimates whether or not the size of detected polyp is the predetermined size or more using AI. Then, the image processing device 1 generates a display image including an endoscopic image, an estimation result of the polyp size, or the like.

The display device 2 is a display or the like for displaying images on the basis of the display signal supplied from the image processing device 1.

The endoscope 3 mainly includes an operation unit 36 used by the examiner to input instructions such as air supply, water supply, angle adjustment, and the photographing instruction, a shaft 37 having flexibility and inserted into an organ of a subject to be examined, a tip portion 38 with a built-in image-taking unit such as an ultra-compact imaging element, and a connection unit 39 for connection with the image processing device 1.

While the following explanation is mainly given on the processing of endoscopic examination for a large intestine, the subjects of examination may be gastrointestinal (digestive organs) such as the stomach, esophagus, small intestine, and duodenum, as well as the large intestine.

### [Hardware configuration]

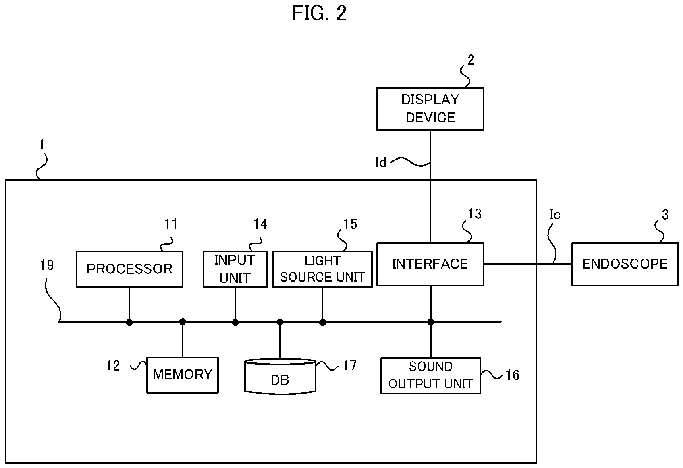

FIG. 2 shows a hardware configuration of the image processing device 1. The image processing device 1 mainly includes a processor 11, a memory 12, an interface 13, an input unit 14, a light source unit 15, a sound output unit 16, and a data base (hereinafter referred to as "DB") 17. These elements are connected with each other via a data bus 19.

The processor 11 executes a predetermined processing by executing a program stored in the memory 12. The processor 11 is a processor such as a CPU (Central Processing Unit), a GPU (Graphics Processing Unit), and a TPU (Tensor Processing Unit). The processor 11 may be configured by multiple processors. The processor 11 is an example of a computer.

The memory 12 is configured by various volatile memories used as a working memory and non-volatile memories for storing information needed for the processing of the image processing device 1, such as a RAM (Random Access Memory) and a ROM (Read Only Memory). Incidentally, the memory 12 may include an external storage device such as a hard disk connected to or incorporated in the image processing device 1, and may include a storage medium such as a removable flash memory or a disk medium. The memory 12 stores a program for the image processing device 1 to execute processing in the present example embodiment.

Also, the memory 12 temporarily stores a series of endoscopic videos Ic taken by the endoscope 3 in the endoscopic examination, based on the control of the processor 11. Further, the memory 12 temporarily stores the still images acquired from the endoscopic video Ic during endoscopic examination. These images are stored in the memory 12 in association with, for example, subject identification information (e.g., the patient ID) and time stamp information, etc.

The interface 13 performs an interface operation between the image processing device 1 and the external devices. For example, the interface 13 supplies the display data Id generated by the processor 11 to the display device 2. Also, the interface 13 supplies the illumination light generated by the light source unit 15 to the endoscope 3. Also, the interface 13 supplies an electrical signal indicating the endoscopic video Ic supplied from the endoscope 3 to the processor 11. The interface 13 may be a communication interface such as a network adapter for wired or wireless communication with an external device, or may be a hardware interface compliant with a USB (Universal Serial Bus), SATA (Serial Advanced Technology Attachment), etc.

The input unit 14 generates an input signal based on the operation of the examiner. The input unit 14 is, for example, a button, a touch panel, a remote controller, a voice input device, or the like. The light source unit 15 generates the light to be delivered to the tip portion 38 of the endoscope 3. The light source unit 15 may also incorporate a pump or the like for delivering water or air to be supplied to the endoscope 3. The sound output unit 16 outputs the sound based on the control of the processor 11.

The DB 17 stores the endoscopic images acquired by past endoscopic examinations of the subject, and lesion information. The lesion information includes lesion image and related information. The lesion includes the polyp (elevated lesion). The DB 17 may include an external storage device, such as a hard disk connected to or incorporated in the image processing device 1, and may include a storage medium, such as a removable flash memory. Instead of providing the DB 17 in the endoscopic examination system 100, the DB 17 may be provided in an external server or the like to acquire associated information from the server through communication.

### [Functional configuration]

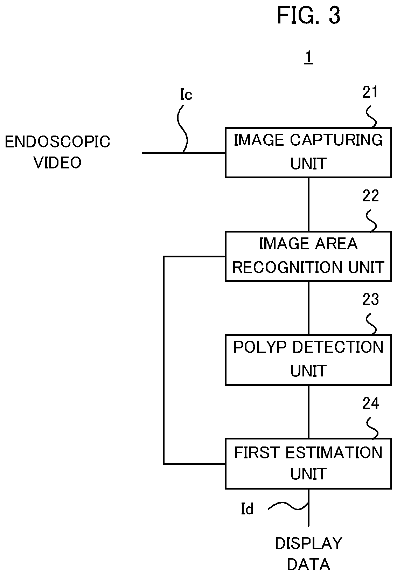

FIG. 3 is a block diagram showing a functional configuration of the image processing device 1. The image processing device 1 functionally includes an image capturing unit 21, an image area recognition unit 22, a polyp detection unit 23, and a first estimation unit 24.

The image processing device 1 receives the endoscopic video Ic from the endoscope 3. The endoscopic video Ic is inputted into the image capturing unit 21. The image capturing unit 21 extracts the still image (frame image) for each frame from the endoscopic video Ic. Here, the extracted frame image includes an area for displaying information about the subject and an area for displaying the image of the endoscope camera. The image capturing unit 21 outputs the extracted frame image to the image area recognition unit 22.

The image area recognition unit 22 recognizes the area for displaying the image of the endoscope camera from the frame image generated by the image capturing unit 21 and cuts out only the area. The image area recognition unit 22 outputs the cut-out image of the area for displaying the image of the endoscope camera (hereinafter, referred to as "endoscopic image") to the polyp detection unit 23 and the first estimation unit 24.

The polyp detection unit 23 detects the polyp from the endoscopic image generated by the image area recognition unit 22, and estimates the position of the polyp. Specifically, the polyp detection unit 23 resizes the endoscopic image generated by the image area recognition unit 22 to the size capable of image analysis by AI. The polyp detection unit 23 detects the polyp included in the resized image by using an image recognition model prepared in advance. This image recognition model is a model which is learned in advance so as to estimate the position of the polyp included in the endoscopic image, and is also called " polyp detection model" hereafter. When the polyp detection unit 23 detects the polyp, the polyp detection unit 23 generates coordinate information of rectangle surrounding the polyp area and outputs the coordinate information to the first estimation unit 24.

The first estimation unit 24 estimates whether or not the polyp detected by the polyp detection unit 23 is equal to or larger than the predetermined size. Specifically, first, the first estimation unit 24 cuts out only the polyp area from the endoscopic image generated by the image area recognition unit 22 using the coordinate information generated by the polyp detection unit 23. Next, the first estimation unit 24 resizes the image of the cut-out polyp area to a size capable of image analysis by AI. Then, the first estimation unit 24 estimates whether or not the polyp is equal to or larger than the predetermined size by using an image recognition model prepared in advance. The image recognition model is a model which is learned in advance so as to estimate whether or not the size of polyp included in the image is equal to or larger than the predetermined size from the image of the polyp area, and is also called "first size estimation model" hereafter.

Next, the first estimation unit 24 estimates whether or not the polyp is equal to or larger than the predetermined size using the first size estimation model. Now, if the predetermined size is set to "Xmm", a polyp of Xmm or largr is set to a large polyp, and a polyp smaller than Xmm is set to a small polyp. Here, the predetermined size "Xmm" is a value which is determined in advance based on guidelines for endoscopic examination and the like. The first estimation unit 24 estimates the size of the polyp included in the image of the polyp area, and calculates a score indicating the probability that the polyp is the large polyp (referred to as a "large polyp score") and a score indicating the probability that the polyp is the small polyp (referred to as a "small polyp score"). For example, the first estimation unit 24 calculates each score so that the sum of the large polyp score and the small polyp score is "1".

Then, the first estimation unit 24 compares the large polyp score and the small polyp score with a predetermined threshold TH, and adopts the score that is larger than the threshold TH as the estimation result. For example, assuming that the threshold TH is "0.5", the first estimation unit 24 estimates that the polyp is the large polyp (Xmm or larger) when the large polyp score is larger than the threshold TH, and the first estimation unit 24 estimates that the polyp is the small polyp (smaller than Xmm) when the small polyp score is larger than the threshold TH. The first estimation unit 24 generates the display data Id based on the endoscopic images and the estimation result of the polyp size and outputs the display data Id to the display device 2.

As described above, in the present example embodiment, when estimating the polyp size based on the endoscopic image, the first estimation unit 24 can estimate the polyp size with high accuracy by using the image narrowed down the polyp area. In the above-described example, the first estimation unit 24 estimates the polyp size after cutting out the polyp area from the endoscopic image. Instead, the first estimation unit 24 may draw the rectangle surrounding the polyp area on the endoscopic image using the coordinate information of the polyp area detected by the polyp detection unit 23, and estimate the polyp size based on the image of the area surrounded by the rectangle.

The first estimation unit 24 may perform drawing that surrounds the polyp area on the endoscopic image with the rectangle or the like, and output the image data to the display device 2. This allows the doctor to view the display image and easily grasp the position of the polyp.

In the above-described configuration, the image capturing unit 21 and the image area recognition unit 22 are example of an endoscopic image acquisition means, the polyp detection unit 23 is an example of a polyp detection means, and the first estimation unit 24 is an example of a first estimation means, an output means, and a display control means.

### [Image display processing]

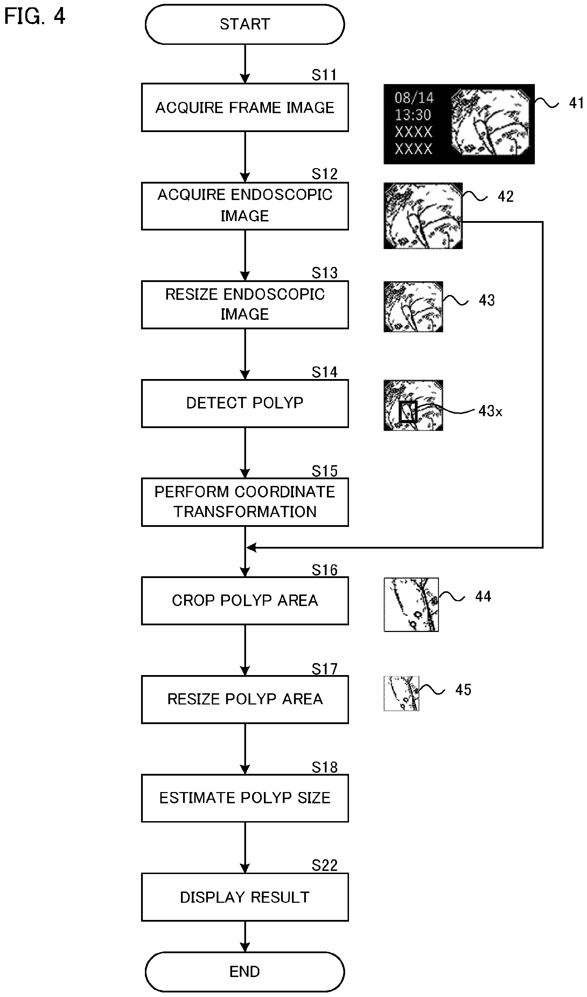

Next, image display processing for performing the above-described display will be described. FIG. 4 is a flowchart of image display processing by the image processing device 1. This processing is realized by the processor 11 shown in FIG. 2, which executes a pre-prepared program and operates as each element shown in FIG. 3.

First, the image capturing unit 21 acquires the endoscopic video Ic through the input unit 14, and acquires the frame image 41 from the endoscopic video Ic (step S11). As shown, the frame image 41 includes the area for displaying information about the subject and the area for displaying the image of the endoscope camera (endoscopic image). The image area recognition unit 22 acquires the endoscopic image 42 from the frame image 41 acquired by the image capturing unit 21 (step S12).

Next, the polyp detection unit 23 resizes the endoscopic image 42 to the suitable size for the polyp detection by the polyp detection model, and generates the resized image 43 (step S13). Next, the polyp detection unit 23 detects the polyp from the resized image 43 using the polyp detection model (step S14). When the polyp detection unit 23 detects the polyp, the polyp detection unit 23 generates the coordinate information of the rectangle 43x indicating the polyp area of the resized image 43. The coordinates of the rectangle 43x indicating the polyp area can be expressed, for example, by the coordinates (x, y) of the upper left point of the rectangle, and the width "w" and height "h" of the rectangle when the point is set as the origin.

Next, the polyp detection unit 23 performs coordinate transformation of the rectangle 43x indicating the polyp area (step S15). The coordinate transformation modifies the coordinates of the rectangle 43x to the coordinates in the coordinate system prior to resizing in the step S13. This modifies the rectangle 43x after coordinate conversion to the size in the coordinate system of the endoscopic image 42. The polyp detection unit 23 outputs the coordinate information of the polyp area after the coordinate transformation to the first estimation unit 24.

Next, the first estimation unit 24 crops the polyp area from the endoscopic image 42 based on the coordinate information of the rectangle 43x indicating the polyp area (step S16). The term crop refers to cutting out a part of an image.

Next, the first estimation unit 24 resizes the cropped polyp area 44 and generates the resized image 45 (step S17). The resizing here modifies the polyp area 44 to the suitable size for the first size estimation model used by the first estimation unit 24. Next, the first estimation unit 24 estimates the polyp size based on the resized image 45 using the first size estimation model (step S18). Specifically, the first estimation unit 24 calculates the score indicating the probability that the polyp is equal to or larger than Xmm (large polyp score) and the score indicating the probability that the polyp is smaller than Xmm (small polyp score). Next, the first estimation unit 24 compares the calculated scores with the threshold TH, and displays the estimation result indicating whether or not the polyp is equal to or larger than Xmm (step S22). Then, the image display processing ends.

### [Display example]

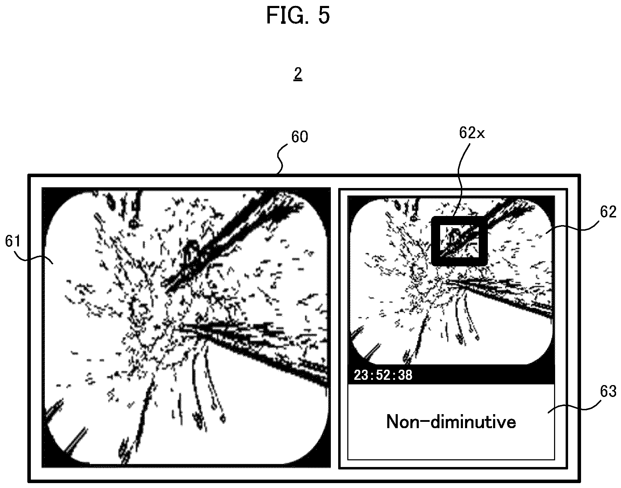

Next, a display example by the display device 2 will be described. FIG. 5 shows a display example of the estimation result of polyp size. At the time of endoscopic examination of a patient, when a polyp is detected, the endoscopic image, the detection result of the polyp, the estimation result of polyp size are displayed on the display device 2.

In the display example of FIG. 5, the endoscope image 61, the polyp detection image 62, and the estimation result of polyp size 63 are displayed in the display area 60.

The endoscope image 61 is the endoscopic image of the organ site where the polyp was detected. The polyp detection image 62 is the image displaying the polyp detected by the polyp detection unit 23, and shows the polyp reflected in the endoscopic image by surrounding with rectangle 62x. In this example, the polyp is surrounded by the rectangle, but it may be surrounded by other figures such as an ellipse. The estimation result of polyp size 63 is the estimation result of polyp size by the first estimation unit 24. In this instance, the word "Non-diminutive" is displayed when the polyp size is equal to or larger than the predetermined size (Xmm), and the word "Diminutive" is displayed when the size of the polyp is smaller than the predetermined threshold. In addition to whether or not the polyp size is equal to or larger than the predetermined size, other information related to the polyp such as the grade of malignancy of the polyp may be displayed in the estimation result of polyp size 63.

### [Modification]

Next, a modification of the first example embodiment will be described. The following modification can be applied to the first example embodiment.

### (Modification 1)

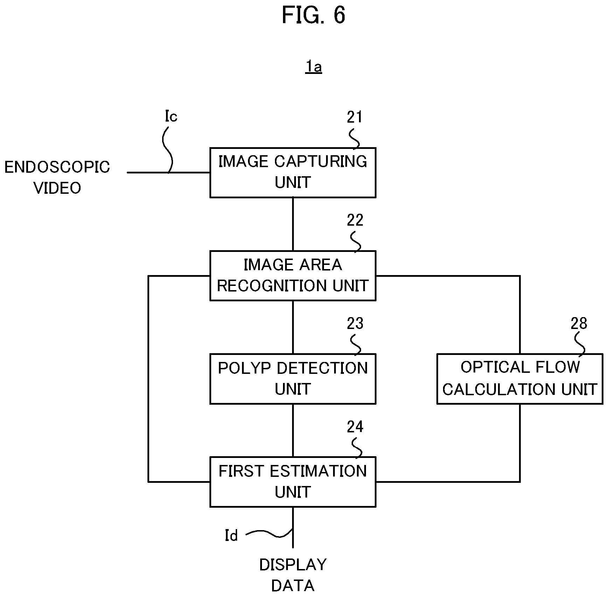

In the first example embodiment, the first estimation unit 24 estimates the polyp size based on the image of the polyp area. Instead, the first estimation unit 24 may estimate the polyp size using optical flow data in addition to the image of the polyp area. FIG. 6 is a block diagram showing a functional configuration of an image display device 1a of the modification 1. As shown, in the modification 1, an optical flow calculation unit 28 is provided on the image display device 1a. The optical flow calculation unit 28 acquires the endoscopic image from the image area recognition unit 22, and calculates the optical flow information based on one frame preceding endoscopic image and the latest endoscopic image. Then, the optical flow calculation unit 28 outputs the calculated optical flow information to the first estimation unit 24. The optical flow calculation unit is an example of a calculation means.

The first size estimation model used by the first estimation unit 24 is the learned model which is learned in advance so as to estimate the polyp size based on the image of the polyp area and the optical flow information. The first estimation unit 24 estimates the polyp size based on the image of the polyp area inputted by the polyp detection unit 23 and the optical flow information calculated by the optical flow calculation unit 28. Since the optical flow indicates the movement direction and movement amount of each point in the image, the accuracy of the estimation of the polyp size can be improved by using the optical flow.

### (Modification 2)

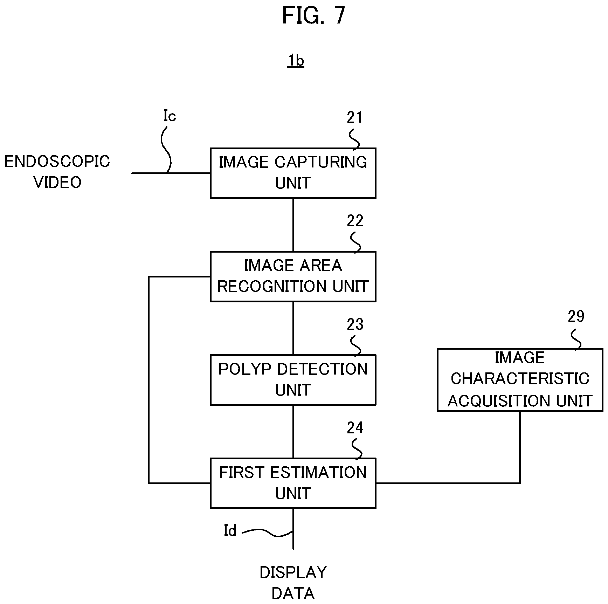

In the estimation of the polyp size, the accuracy of the estimation of the polyp size can be improved by considering the manufacturer of the endoscope, the type of light source used by the endoscope, whether or not the endoscope performs enlarged display, and the like. FIG. 7 is a block diagram showing a functional configuration of an image processing device 1b of the modification 2. As shown, in the modification 2, an image characteristic acquisition unit 29 is provided on the image processing device 1b. The image characteristic acquisition unit 29 acquires image characteristic information such as the manufacturer of the endoscope, the type of light source used by the endoscope, whether or not the endoscope performs enlarged display, and the like. Specifically, the specification information of the endoscope may be used as the image characteristic information. In this case, the image characteristic acquisition unit 29 may read the specification information from a storage medium or the like that stores the specification information of the endoscope. Instead, the image characteristic acquisition unit 29 may estimate the image quality such as the brightness and the color of the image by analyzing the endoscopic image generated by the image area recognition unit 22, and use it as the image characteristic information. The image characteristic acquisition unit 29 is an example of an image characteristic acquisition means.

The image characteristic acquisition unit 29 outputs the extracted image characteristic information to the first estimation unit 24. The first size estimation model used by the first estimation unit 24 is the learned model which is learned in advance so as to estimate the polyp size based on the image of the polyp area and the image characteristic information. The first estimation unit 24 estimates the polyp size based on the image of the polyp area inputted by the polyp detection unit 23 and the image characteristic information inputted by the image characteristic acquisition unit 29. This makes it possible to improve the accuracy of the estimation of the polyp size.

### <Second example embodiment>

Next, a second example embodiment of the present invention will be described. Since the configuration of the endoscopic examination system 100 and hardware configuration of the image processing device 1x according to the second example embodiment are the same as those in the first example embodiment described above, the description thereof will not be repeated.

### [Functional configuration]

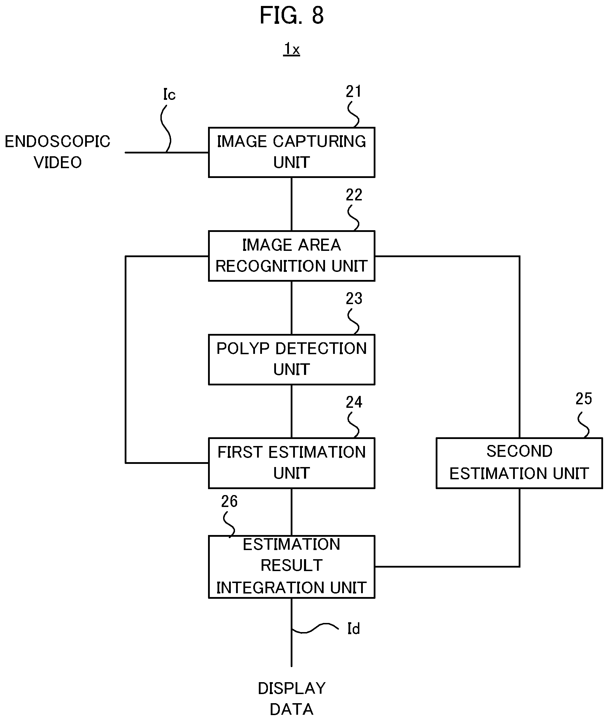

FIG. 8 is a block diagram showing a functional configuration of the image processing device 1x according to the second example embodiment. The image processing device 1x according to the second example embodiment is obtained by adding a second estimation unit 25 and an estimation result integration unit 26 to the image processing device 1 according to the first example embodiment.

The second estimation unit 25 estimates the polyp size from the endoscopic image generated by the image area recognition unit 22 using a second size estimation model. That is, the first size estimation model estimates the polyp size from the image of the polyp area, whereas the second size estimation model estimates the polyp size from the entire endoscopic image. The second size estimation model is the model which is learned in advance so as to estimate the size of the polyp included in the endoscopic image.

Specifically, first, the second estimation unit 25 resizes the endoscopic image generated by the image area recognition unit 22 to the suitable size for the second size estimation model. Next, the second estimation unit 25 detects the polyp using the second size estimation model prepared in advance and estimates whether or not the polyp is equal to or larger than the predetermined size. Similar to the first estimation unit 24, the second estimation unit 25 calculates the score indicating the probability that the polyp included in the endoscopic image is equal to or larger than Xmm (large polyp score) and the score indicating the probability that the polyp included in the endoscopic image is smaller than Xmm (small polyp score), and outputs the score to the estimation result integration unit 26.

The estimation result integration unit 26 integrates the estimation result of the polyp size by the first estimation unit 24 and the estimation result of the polyp size by the second estimation unit 25. For example, regarding the size of a certain polyp, it is assumed that the first estimation unit 24 outputs the large polyp score "0.6" and the small polyp score "0.4", and the second estimation unit 25 outputs the large polyp score "0.8" and the small polyp score "0.2". First, the estimation result integration unit 26 calculates the average value of the estimation results of the first estimation unit 24 and the second estimation unit 25. In this example, the estimation result integration unit 26 calculates the average value of the large polyp score as "0.7" and the average value of the small polyp score as "0.3". Then, the estimation result integration unit 26 compares the obtained average value with a predetermined threshold TH and outputs the estimation result of the polyp size. As described above, assuming that the threshold TH is "0.5", the estimation result integration unit 26 estimates that the polyp is a large polyp, that is, the polyp size is Xmm or larger. Then, the estimation result integration unit 26 generates the display data Id based on the estimation result of the polyp size and the endoscopic image generated by the image area recognition unit 22, and outputs the display data Id to the display device 2.

As described above, in the second example embodiment, the second estimation unit 25 that estimates the polyp size from the entire endoscopic image is added to the configuration of the first example embodiment. Thus, if the polyp detection unit 23 misses the polyp, the second estimation unit 25 is possible to supplement it by detecting the polyp from the entire endoscope image and estimating the polyp size.

In the above-described configuration, the second estimation unit 25 is an example of a second estimation unit, and the estimation result integration unit 26 is an example of an estimation result integration means.

### [Image display processing]

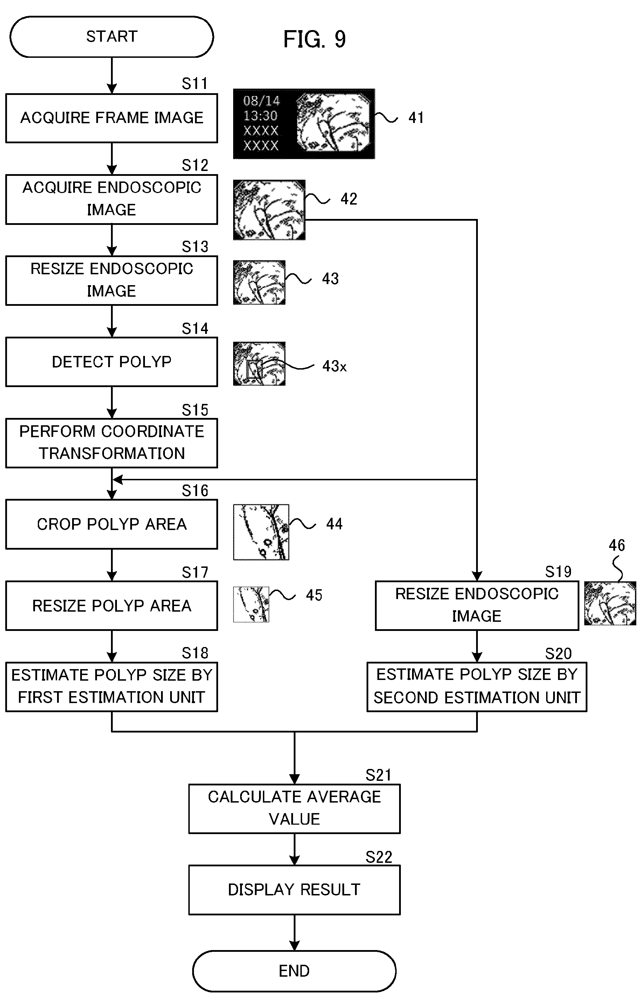

FIG. 9 is a flowchart of image display processing by the image processing device 1x according to the second example embodiment. The image display processing flowchart according to the second example embodiment is obtained by adding the step S19-S21 to the image display processing flowchart according to the first example embodiment shown in FIG. 4. Since the processing of step S11~S18 is the same as that in the first example embodiment, the description thereof will not be repeated.

The second estimation unit 25 acquires the endoscopic image 42 generated by the image area recognition unit 22 in the step S12. Then, the second estimation unit 25 resizes the endoscopic image 42 to the suitable size for the second size estimation model to generate the resized image 46 (step S19). Next, the second estimation unit 25 detects the polyp based on the resized image 46 using the second size estimation model, and outputs the estimation result of the polyp size to the estimation result integration unit 26 (step S20). The estimation result integration unit 26 calculates the average value of the size estimation result of the first estimation unit 24 and the size estimation result of the second estimation unit 25 (step S21), and determines the estimation result of the polyp size by comparing the average value with the threshold TH and displays it (step S22). Then, the processing ends.

### [Modification]

Next, a modification of the second example embodiment will be described. The following modification can be applied to the second example embodiment.

### (Modification 3)

In the second example embodiment, the first estimation unit 24 estimates the polyp size based on the image of the polyp area, and the second estimation unit 25 estimates the polyp size based on the endoscopic image. Here, the second estimation unit 25 may be configured to estimate the polyp size using the optical flow information. In this case, similarly to the modification 1 described above, the optical flow calculation unit 28 is provided on the image processing device 1x, and the optical flow information calculated by optical flow calculation unit 28 is inputted into the second estimation unit 25. The second size estimation model used by the second estimation unit 25 is the learned model which is learned in advance so as to estimate the polyp size based on the endoscopic image and the optical flow information.

Instead of the second estimation unit 25, the first estimation unit 24 may be configured to estimate the polyp size using the optical flow information. In addition, both the first estimation unit 24 and the second estimation unit 25 may be configured to estimate the polyp size using the optical flow information. Furthermore, apart from the first estimation unit 24 and the second estimation unit 25, an estimation unit for estimating the polyp size based on the endoscope image and the optical flow information may be provided. Thus, similarly to the modification 1, it is possible to improve the accuracy of the polyp size estimation using the optical flow information.

### (Modification 4)

Also in the second example embodiment, in the estimation of the polyp size, the accuracy of the estimation of the polyp size can be improved by considering the manufacturer of the endoscope, the type of light source used by the endoscope, whether or not the endoscope performs enlarged display, and the like. In this case, similarly to modification 2 described above, the image characteristic acquisition unit 29 is provided on the image processing device 1x, and the image characteristic information acquired by the image characteristic acquisition unit 29 is inputted to the second estimation unit 25. The second size estimation model used by the second estimation unit 25 is the model which is learned in advance so as to estimate the polyp size based on the endoscopic image and the image characteristic information. Thus, similarly to the modification 2, it is possible to improve the accuracy of the polyp size estimation using the image characteristic information. Instead of the second estimation unit 25, the first estimation unit 24 may be configured to estimate the polyp size using the image characteristic information. In addition, both the first estimation unit 24 and the second estimation unit 25 may be configured to estimate the polyp size using the image characteristic information.

### <Third example embodiment>

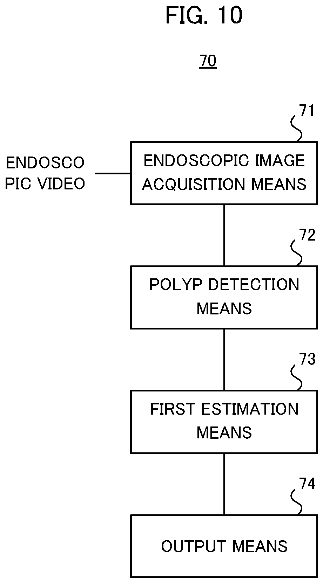

FIG. 10 is a block diagram showing a functional configuration of an image processing device of a third example embodiment. The image processing device 70 includes an endoscopic image acquisition means 71, a polyp detection means 72, a first estimation means 73, and an output means 74.

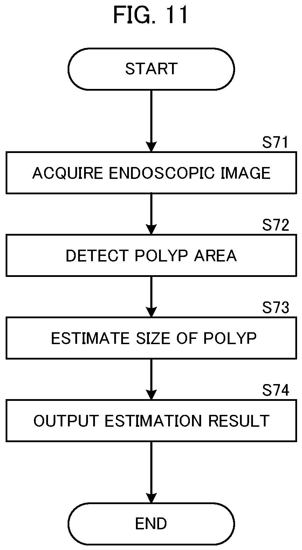

FIG. 11 is a flowchart of processing by the image processing device of the third example embodiment. The endoscopic image acquisition means 71 acquires an endoscopic image (step S71). The polyp detection means 72 detects a polyp area from the endoscopic image (step S72). The first estimation means 73 estimates a size of the polyp based on an image of the detected polyp area (step S73). The output means 74 outputs an estimation result of the size of the polyp (step S74).

According to the image processing device 70 of the third example embodiment, it is possible to detect the polyp during endoscopic examination and estimate the size of the polyp based on the image of the polyp area.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

### (Supplementary note 1)

An image processing device comprising:
an endoscopic image acquisition means configured to acquire an endoscopic image;
a polyp detection means configured to detect a polyp area from the endoscopic image;
a first estimation means configured to estimate a size of the polyp based on an image of the detected polyp area; and
an output means configured to output an estimation result of the size of the polyp.

### (Supplementary note 2)

The image processing device according to Supplementary note 1, wherein the first estimation means cuts out the image of the polyp area from the endoscope image and estimates the size of the polyp based on the image of the cut out polyp area, when the polyp detection means detects the polyp.

### (Supplementary note 3)

The image processing device according to Supplementary note 1, wherein the first estimation means performs drawing on the endoscope image so as to surround the polyp area and estimates the size of the polyp based on the surrounded polyp area, when the polyp detection means detects the polyp.

### (Supplementary note 4)

The image processing device according to any one of Supplementary notes 1 to 3, further comprising a display control means configured to display the endoscopic image and an estimation result of the size of the polyp on a display device,
wherein the display control means superimposes and displays a figure surrounding the detected polyp area on the endoscopic image.

### (Supplementary note 5)

The image processing device according to any one of Supplementary notes 1 to 4, further comprising a calculation means configured to calculate optical flow information based on the endoscopic image,
wherein the first estimation means estimates the size of the polyp based on the image of the polyp area and the optical flow information.

### (Supplementary note 6)

The image processing device according to any one of Supplementary notes 1 to 5, further comprising an image characteristic acquisition means configured to acquire image characteristic information of the endoscopic image,
wherein the first estimation means estimates the size of the polyp based on the image of the polyp area and the image characteristic information.

### (Supplementary note 7)

The image processing device according to any one of Supplementary notes 1 to 6, further comprising a second estimation means configured to estimate the size of the polyp based on the endoscopic image; and
an estimation result integration means configured to integrate the estimation result of the size of the polyp by the first estimation means and the estimation result of the size of the polyp by the second estimation means,
wherein the output means outputs an integrated result by the estimation result integration means.

### (Supplementary note 8)

The image processing device according to Supplementary note 7, further comprising a calculation means configured to calculate optical flow information based on the endoscopic image,
wherein the second estimation means estimates the size of the polyp based on the endoscope image and the optical flow information.

### (Supplementary note 9)

The image processing device according to any one of Supplementary notes 7 to 8, further comprising an image characteristic acquisition means configured to acquire image characteristic information of the endoscopic image,
wherein the second estimation means estimates the size of the polyp based on the endoscope image and the image characteristic information.

### (Supplementary note 10)

An image processing method comprising:
acquiring an endoscopic image;
detecting a polyp area from the endoscopic image;
estimating a size of the polyp based on an image of the detected polyp area; and
outputting an estimation result of the size of the polyp.

### (Supplementary note 11)

A recording medium storing a program, the program causing a computer to execute processing of:
acquiring an endoscopic image;
detecting a polyp area from the endoscopic image;
estimating a size of the polyp based on an image of the detected polyp area; and
outputting an estimation result of the size of the polyp.

### DESCRIPTION OF SYMBOLS

1 Image processing device
2 Display device
3 Endoscope
11 Processor
12 Memory
17 Database (DB)
21 Image capturing unit
22 Image area recognition unit
23 Polyp detection unit
24 First estimation unit
25 Second estimation unit
26 Estimation result integration unit
100 Endoscopic examination system

## Claims

1. An image processing device comprising:
an endoscopic image acquisition means configured to acquire an endoscopic image;
a polyp detection means configured to detect a polyp area from the endoscopic image;
a first estimation means configured to estimate a size of the polyp based on an image of the detected polyp area; and
an output means configured to output an estimation result of the size of the polyp.

2. The image processing device according to claim 1, wherein the first estimation means cuts out the image of the polyp area from the endoscope image and estimates the size of the polyp based on the image of the cut out polyp area, when the polyp detection means detects the polyp.

3. The image processing device according to claim 1, wherein the first estimation means performs drawing on the endoscope image so as to surround the polyp area and estimates the size of the polyp based on the surrounded polyp area, when the polyp detection means detects the polyp.

4. The image processing device according to any one of claims 1 to 3, further comprising a display control means configured to display the endoscopic image and an estimation result of the size of the polyp on a display device,
wherein the display control means superimposes and displays a figure surrounding the detected polyp area on the endoscopic image.

5. The image processing device according to any one of claims 1 to 4, further comprising a calculation means configured to calculate optical flow information based on the endoscopic image,
wherein the first estimation means estimates the size of the polyp based on the image of the polyp area and the optical flow information.

6. The image processing device according to any one of claims 1 to 5, further comprising an image characteristic acquisition means configured to acquire image characteristic information of the endoscopic image,
wherein the first estimation means estimates the size of the polyp based on the image of the polyp area and the image characteristic information.

7. The image processing device according to any one of claims 1 to 6, further comprising a second estimation means configured to estimate the size of the polyp based on the endoscopic image; and
an estimation result integration means configured to integrate the estimation result of the size of the polyp by the first estimation means and the estimation result of the size of the polyp by the second estimation means,
wherein the output means outputs an integrated result by the estimation result integration means.

8. The image processing device according to claim 7, further comprising a calculation means configured to calculate optical flow information based on the endoscopic image,
wherein the second estimation means estimates the size of the polyp based on the endoscope image and the optical flow information.

9. The image processing device according to any one of claims 7 to 8, further comprising an image characteristic acquisition means configured to acquire image characteristic information of the endoscopic image,
wherein the second estimation means estimates the size of the polyp based on the endoscope image and the image characteristic information.

10. An image processing method comprising:
acquiring an endoscopic image;
detecting a polyp area from the endoscopic image;
estimating a size of the polyp based on an image of the detected polyp area; and
outputting an estimation result of the size of the polyp.

11. A recording medium storing a program, the program causing a computer to execute processing of:
acquiring an endoscopic image;
detecting a polyp area from the endoscopic image;
estimating a size of the polyp based on an image of the detected polyp area; and
outputting an estimation result of the size of the polyp.
